(19) Europäisches Patentamt
European Patent Office
Office européen des brevets

(11) EP 0 689 837 B1

(12) **EUROPÄISCHE PATENTSCHRIFT**

(45) Veröffentlichungstag und Bekanntmachung des Hinweises auf die Patenterteilung:
**12.12.2001 Patentblatt 2001/50**

(51) Int Cl.$^7$: **A61K 31/505**

(21) Anmeldenummer: **95109812.8**

(22) Anmeldetag: **23.06.1995**

(54) **Antihyperglykämisch wirksame Arzneimittel**

Anti-hyperglycemic medicaments

Médicaments anti-hyperglycémiques

(84) Benannte Vertragsstaaten:
**AT BE CH DE DK ES FR GB GR IE IT LI LU NL PT SE**

(30) Priorität: **01.07.1994 DE 4423177**

(43) Veröffentlichungstag der Anmeldung:
**03.01.1996 Patentblatt 1996/01**

(73) Patentinhaber: **Solvay Pharmaceuticals GmbH**
**30173 Hannover (DE)**

(72) Erfinder:
- **Kaan, Elbert, Dr.**
  **D-30938 Grossburgwedel (DE)**
- **Ziegler, Dieter, Dr.**
  **D-30966 Hemmingen (DE)**
- **Brückner, Reinhard, Dr.**
  **D-30559 Hannover (DE)**

(74) Vertreter: **Lauer, Dieter, Dr.**
**Solvay Pharmaceuticals GmbH,**
**Hans-Böckler-Allee 20**
**30173 Hannover (DE)**

(56) Entgegenhaltungen:
**EP-A- 0 488 897** **DE-A- 2 849 537**

- **THERAPIEWOCHE, Bd. 43, Nr. 42-43, 1993 Seiten 1686-1693, XP 000567122 RUPP ET AL. 'Antihypertensiva bei Hyperinsulinämie. Durch Sympathikus-Regulation ans Ziel'**
- **Z. ALLG. MED., Bd. 68, 1992 Seiten 760-763, XP 000567127 DOTZER ET AL. 'Moxonidin bei Hypertonikern mit Begleiterkrankungen breit einsetzbar'**
- **J. CARDIOVASC. PHARMACOL., Bd. 20, Nr. 4, 1992 Seiten s24-s30, XP 000567125 MICHEL ET AL. 'From alpha2-adrenoceptors to imidazoline receptors: putative progress for cardiovascular therapy'**
- **MOL. CELL. BIOCHEM., Bd. 132, Nr. 1, 16.März 1994 Seiten 69-80, XP 000567154 RUPP ET AL. 'Modification of myosin isozymes and SR Ca(2+)-pump ATPase of the diabetic rat heart by lipid-lowering interventions'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft die Verwendung von 4-Chlor-5-[(4,5-dihydro-1H-imidazol-2-yl)-amino]-6-methoxy-2-methylpyrimidin (= Moxonidin) und dessen physiologisch verträglichen Säureadditionssalzen zur Behandlung von Hyperglykämien, und zur Herstellung von für diese Behandlung geeigneten Arzneimitteln.

Erfindungsgemäß werden zur Herstellung von pharmazeutischen Zubereitungen zur Behandlung von Hyperglykämien 4-Chlor-5-[(4,5-dihydro-1H-imidazol-2-yl)-amino]-6-methoxy-2-methylpyrimidin der Formel I

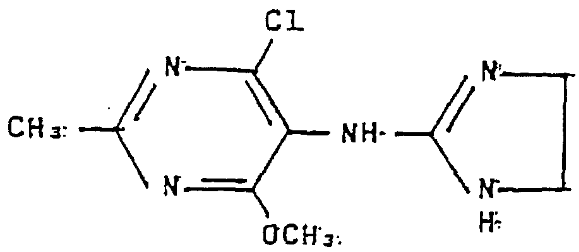

I

und dessen physiologisch verträgliche Säureadditionssalze verwendet.

Als physiologisch verträgliche Säureadditionssalze des Moxonidins eignen sich Salze mit anorganischen Säuren, beispielsweise Halogenwasserstoffsäuren, oder mit organischen Säuren, beispielsweise niederen aliphatischen Mono- oder Dicarbonsäuren wie Essigsäure, Fumarsäure oder Weinsäure oder aromatischen Carbonsäuren wie z. B. Salicylsäure.

Die erfindungsgemäß zur Behandlung von hyperglykämischen Zuständen eingesetzten Verbindungen fallen unter den Umfang von in der deutschen Offenlegungsschrift DE-A-28 49 537 beschriebenen 5-[(2-Imidazolin-2-yl)-amino]-pyrimidin-Derivaten mit blutdrucksenkenden Eigenschaften, und sind aus dieser Patentanmeldung bekannt. Moxonidin-haltige pharmazeutische Zubereitungen sind als Antihypertensiva unter dem Markennamen Physiotens$^R$ im Handel erhältlich und werden medizinisch als Antihypertensivum eingesetzt. Die Verbindungen können auf an sich bekannte Weise nach den in der vorgenannten DE-A-28 49 537 beschriebenen Verfahren oder analog zu diesen Verfahren hergestellt werden.

Es ist im Stand der Technik bekannt, daß ein hoher Prozentsatz von Hypertonikern eine Glucoseintoleranz als Folge verminderter Insulinsensitivität aufweist. In der Literatur (Rupp et al., Therapiewoche 43 (1993), Seiten 1686 - 1693) wird daher diskutiert, daß die Normalisierung des hohen Blutdruckes nicht das einzige Therapieziel sein kann, sondern daß die mit Bluthochdruck oftmals einhergehenden metabolischen Störungen mitbehandelt werden sollten. Dotzer et al. (Z. Allg. Med., 1992, 68: 760 - 763) und Michel et al. (J. Cardiovasc. Pharmacol., 1992, 20: 24 - 30) bestätigt dieses und stellen fest, daß Moxonidin bei Hypertonikern mit Begleiterkrankungen breit einsetzbar ist, ohne die metabolischen Begleiterkrankungen der essentiellen Hypertonie ungünstig zu beeinflussen. Rupp et al. (Moll. Cell. Biochem., 1994; 132: 69 - 80) untersuchten Etomoxir und daneben weitere Substanzen wie u. a. Moxonidin an diabetischen Ratten, um die Rolle des adrenergen Einflusses zu bestimmen. Es wurde festgestellt, daß Moxonidin bei diabetischen Ratten keinen signifikanten Effekt auf Serum-Insulin oder Glucose ausübte. Die EP 488 897 A1 beschreibt allgemein strukturell von Moxonidin verschiedene Guanidin-Verbindungen mit 5-HT-Rezeptor-Affinität, z. B. für die Behandlung von Bluthochdruck, Gefäßerkrankungen, Diabetes oder Fettleibigkeit, ohne jedoch Daten anzugeben. Keines der vorstehenden Dokumente, welche zwar z. B. den Einfluß von Moxonidin und anderen Substanzen auf metabolische Begleiterscheinungen des Bluthochdrucks ansprechen, enthält jedoch Hinweise darauf, daß Moxonidin erhöhte Blutzuckerspiegel positiv beeinflussen kann.

Es wurde nun überraschenderweise gefunden, daß Moxonidin und seine physiologisch verträglichen Säureadditionssalze eine antihyperglykämische Wirkung am Menschen und größeren Säugetieren besitzen und zur Behandlung von mit Hyperglykämie einhergehenden Stoffwechselstörungen verschiedenster Genese geeignet sind, beispielsweise dem Auftreten von erhöhten Plasmaglucosewerten aufgrund erhöhter Glucosefreisetzung und/oder verminderter metabolischer Glucoseverwertung, welche im Zusammenhang mit erhöhtem Blutdruck, Insulinresistenz, Glucoseintoleranz, Typ II Diabetes und/oder Obesitas stehen können.

Zur erfindungsgemäßen Behandlung von hyperglykämischen Zuständen können Moxinidin und seine physiologisch verträglichen Säureadditionssalze in üblichen pharmazeutischen Zubereitungen oral, intravenös oder auch transdermal verabreicht werden.

So können antihyperglykämisch wirksame Mengen der Verbindungen erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese festen Präparate können pharmazeutisch übliche anorganische und/oder organische Trägerstoffe wie z. B. Milchzucker, Talkum oder Stärke neben pharmazeutisch üblichen Hilfsmitteln, beispielsweise Gleitmitteln oder Tablettensprengmitteln, enthalten. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfsund/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die antihyperglykämische Wirkung von Moxonidin wurde in Tierversuchen und in klinischen Studien an Patienten mit unterschiedlichen Graden von Hyperglykämie nachgewiesen.

Es wurde eine Doppelblindstudie mit insgesamt 228 Patienten über einen Zeitraum von 6 Wochen durchgeführt.

Die Patienten wurden nach dem Zufallsprinzip in 4 Gruppen eingeteilt. Alle Patienten hatten je eine Tablette zweimal täglich einzunehmen. In einer Testvorlaufphase von 4 Wochen erhielten alle Patienten Placebotabletten. In der eigentlichen Testphase erhielt eine Kontrollgruppe (= Gruppe K) von Patienten Placebotabletten, eine erste Testgruppe (= Gruppe 1) Tabletten mit einem Gehalt von 0,1 mg Moxonidin pro Tablette, eine zweite Testgruppe (= Gruppe 2) Tabletten mit einem Gehalt von 0,2 mg Moxonidin pro Tablette und eine dritte Testgruppe (= Gruppe 3) Tabletten mit einem Gehalt von 0,4 mg Moxonidin pro Tablette. Den Patienten wurden jeweils in nüchternem Zustand am Tag vor dem Beginn der Testphase und nach 6 Wochen am letzten Tag der Testphase Blutproben entnommen. In diesen wurden die Plasmablutzuckerwerte in mg Glucose pro Deziliter gemessen.

Zur Auswertung der Meßergebnisse wurden für jede der 4 Gruppen eine weitere Unterteilung in je 2 Untergruppen vorgenommen:

A) Probanden mit normalen Anfangsplasmaglucosewerten im Bereich von ≤ 115 mg/dl

B) Probanden mit pathologisch erhöhten Anfangsplasmaglucosewerten von > 115 mg/dl. Diese Untergruppe umfaßt Patienten mit leicht erhöhten Anfangsplasmaglucosewerten im Bereich von 115 bis 139 mg/dl und Patienten mit deutlich erhöhten Anfangsplasmaglucosewerten im Diabetesbereich (≥ 140 mg/dl). Die Meßergebnisse dieser Unteruntergruppen B1) von Diabetespatienten wurden nochmals gesondert ausgewertet.

Die nachfolgende Tabelle gibt für alle Untergruppen die errechneten statistischen Mittelwerte (± Standardfehler) der Plasmablutzuckerbestimmungen an.

**Tabelle:** Änderung der Plasmaglucosewerte

| Medikation | Patientengruppe | Anzahl Patienten | Plasmaglucosewerte in mg/dl (Mittelwerte ± Standardfehler) Anfangswert | Endwert |
|---|---|---|---|---|
| Placebo | K A<br>K B<br>K B 1 | 49<br>9<br>1 | 94 (± 1)<br>134 (± 5)<br>172 | 93 (± 2)<br>129 (± 6)<br>174 |
| 0,1 mg Moxonidin 2 x täglich | 1 A<br>1 B<br>1 B 1 | 52<br>7<br>1 | 95 (± 1)<br>131 (± 8)<br>183 | 93 (± 2)<br>117 (± 4)<br>103 |
| 0,2 mg Moxonidin 2 x täglich | 2 A<br>2 B<br>2 B 1 | 45<br>10<br>6 | 93 (± 2)<br>170 (± 17)<br>198 (± 21) | 95 (± 2)<br>134 (± 8)<br>144 (± 12) |
| 0,4 mg Moxonidin 2 x täglich | 3 A<br>3 B<br>3 B 1 | 46<br>10<br>2 | 92 (± 2)<br>130 (± 5)<br>158 (± 7) | 91 (± 2)<br>120 (± 10)<br>139 (± 28) |

Aus der vorstehenden Tabelle ist ersichtlich, daß während der Testphase bei allen nur mit Placebo behandelten Patienten unabhängig vom Anfangsplasmaglucosewert praktisch keine Veränderung der Blutzuckerwerte auftrat. Bei den mit verschiedenen Dosen von Moxonidin behandelten Patienten zeigte sich, daß bei Patienten mit normalen Anfangsplasmaglucosewerten ebenfalls praktisch keine Veränderung der Plasmaglucosewerte auftrat. Bei Patienten mit erhöhten Anfangsplasmaglucosewerten trat jedoch ein deutlicher Rückgang dieser Plasmaglucosewerte durch die Moxinidin-Behandlung ein, wobei diese Reduktion der Plasmaglucosewerte umso stärker war, je höher die Anfangsplasmaglucosewerte waren.

Die vorstehenden Versuchsergebnisse zeigen, daß Moxonidin eine antihyperglykämische Wirkung ausüben und den Abbau erhöhter Blutzuckerwerte bewirkt, ohne jedoch normale Blutzuckerwerte zu beeinträchtigen. Diese Versuchsergebnisse sind auch als Indiz dafür zu werten, daß Moxonidin einen günstigen Einfluß auf Insulinresistenz hat. Deshalb sind Moxonidin und seine Säureadditionssalze zur Behandlung von Hyperglykämien geeignet. Die zu verwendenden Dosen können individuell verschieden sein und variieren naturgemäß je nach Art des zu behandelnden Zustandes und der Applikationsform. Im allgemeinen eignen sich zur Behandlung von hyperglykämischen Zuständen beim Menschen bei oraler Applikation Tagesdosen im Bereich von 0,2 bis 0,8 mg, vorzugsweise 0,4 bis 0,8 mg.

Das folgende Beispiel soll die Herstellung einer zur Behandlung von Hyperglykämien geeigneten Moxonidin enthaltenden pharmazeutischen Zubereitung näher erläutern, ohne jedoch den Umfang der Anmeldung zu begrenzen.

**Beispiel 1:**

Moxonidin-haltige filmüberzogene Tabletten

**Zusammensetzung:**

| Tablettenkerne: | |
|---|---|
| Moxonidin | 0,020 Teile |
| Lactose | 9,580 Teile |
| Povidone USP | 0,070 Teile |
| Crospovidone USP | 0,300 Teile |
| Magnesiumstearat | 0,030 Teile |
| (Wasser | 0,750 Teile) |

(fortgesetzt)

| Tablettenkerne: | |
|---|---|
| Gesamtfeststoff | 10,000 Teile |

| Filmüberzug: | |
|---|---|
| Hydroxypropylmethylcellulose | 0,156 Teile |
| 30 %-ige wäßrige Ethylcellulose-Dispersion<br>(≙ Feststoff) | 0,480 Teile<br>(0,144) Teile |
| Polyethylenglycol 6000 | 0,030 Teile |
| Titandioxid | 0,150 Teile |
| Talc | 0,1197 Teile |
| Rotes Eisenoxid | 0,0003 Teile |
| (Wasser | 3,864 Teile) |
| Gesamtfeststoff | 0,600 Teile |
| Gesamtmenge an Filmüberzugssuspension | 4,800 Teile |

Zum Überziehen von 10.000 Tablettenkerne zu je 100 mg Gewicht werden 4,8 kg der vorstehenden Filmüberzugssuspension eingesetzt.

**Tablettenkernherstellung:**

Das Moxonidin und die Lactose wurden gemischt. Die Mischung wurde mit einer Lösung des Bindemittels Povidone in Wasser durchfeuchtet, gut durchgeknetet und das erhaltene Produkt wurde auf Horden ausgebreitet und bei einer Temperatur von ca. 50 °C bis zu einem Feuchtigkeitsgehalt von höchstens 0,5 % getrocknet. Das getrocknete Produkt wurde durch ein 0,75 mm-Sieb (Frewitt-Maschine) passiert. Nach dem Vermischen des erhaltenen Granulates mit Crospovidone und Magnesiumstearat wurden daraus Tablettenkerne mit einem Gewicht von 100 mg gepreßt, so daß jeder Tablettenkern 0,2 mg Wirkstoff enthielt.

**Herstellung der Filmüberzugssuspension:**

Die Hydroxypropylmethylcellulose und das Polyethylenglycol 6000 wurden in einem Teil des Wassers gelöst. Zu dieser Lösung wurde eine Suspension von Talc, Titandioxid und Eisenoxid in dem übrigen Wasser unter Rühren zugefügt. Die erhaltene Suspension wurde unter leichtem Rühren mit der 30 %igen wäßrigen Ethylcellulose-Dispersion verdünnt.

**Filmüberziehen der Tablettenkerne:**

Die Filmüberzugssuspension wurde auf die Tablettenkerne in einer Befilmungsapparatur gesprüht, während warme Luft von ca. 70 °C die Tablettenkerne auf eine Temperatur von ca. 45 °C erwärmte. Anschließend wurden die filmüberzogenen Tabletten 16 Stunden bei einer Temperatur von ca. 45 °C getrocknet.

## Patentansprüche

1. Verwendung von 4-Chlor-5-[(4,5-dihydro-1H-imidazol-2-yl)-amino]-6-methoxy-2-methylpyrimidin der Formel I

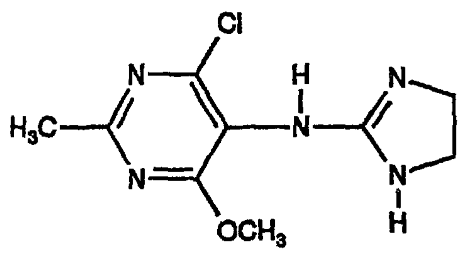

I

und dessen physiologisch verträglichen Säureadditionssalze zur Herstellung von pharmazeutischen Zubereitungen zur Behandlung von Hyperglykämien bei Menschen und größeren Säugetieren.

**Claims**

1. The use of 4-chloro-5-[(4,5-dihydro-1 H-imidazol-2-yl)-amino]-6-methoxy-2-methylpyrimidine of Formula I

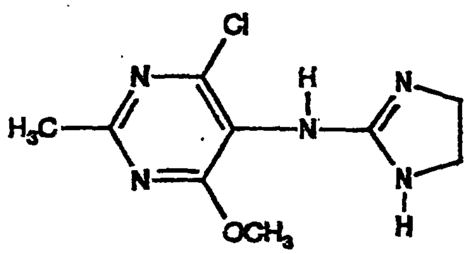

· I

and the physiologically compatible acid addition salts thereof for the preparation of pharmaceutical preparations for the treatment of hyperglycaemia in humans and larger mammals.

**Revendications**

1. Utilisation de la 4-chloro-5-[(4,5-dihydro-lH-imidazole-2-yl)-amino]-6-méthoxy-2-méthylpyrimidine de formule I

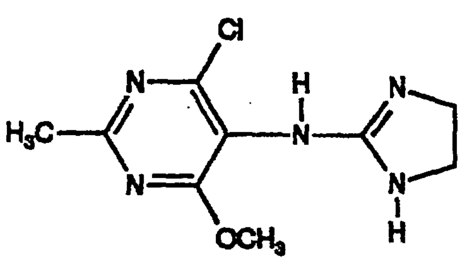

(I)

et de ses sels d'addition avec un acide, physiologiquement tolérés, pour fabriquer des préparations pharmaceutiques servant au traitement des hyperglycémies chez l'homme et les grands mammifères.